Europäisches Patentamt

European Patent Office

Office européen des brevets

(11) Numéro de publication : **0 150 681**
**B1**

## (12) FASCICULE DE BREVET EUROPÉEN

(45) Date de publication du fascicule du brevet :
23.12.87

(21) Numéro de dépôt : **84810661.3**

(22) Date de dépôt : **28.12.84**

(51) Int. Cl.⁴ : **A 61 M   5/28**, A 61 M   5/24

(54) **Seringue préremplie à dose unitaire.**

(30) Priorité : **25.01.84 CH 344/84**

(43) Date de publication de la demande :
**07.08.85 Bulletin 85/32**

(45) Mention de la délivrance du brevet :
**23.12.87 Bulletin 87/52**

(84) Etats contractants désignés :
**AT BE CH DE FR GB IT LI LU NL SE**

(56) Documents cités :
**EP-A- 0 111 796**
**DE-C-   139 640**
**FR-A- 1 484 784**
**US-A- 2 971 509**

(73) Titulaire : **MEDICORP HOLDING S.A.**
**11 Boulevard du Prince Henri**
**L-2014 Luxembourg (LU)**

(72) Inventeur : **Meyer, Gabriel**
**10A, chemin des Princes**
**1222 Vesenaz (CH)**
Inventeur : **Howald, Ernst**
**10B, chemin des Princes**
**1222 Vesenaz (CH)**

(74) Mandataire : **Nithardt, Roland**
**CABINET ROLAND NITHARDT Rue Edouard Verdan 15**
**CH-1400 Yverdon-les-Bains (CH)**

EP 0 150 681 B1

Jouve, 18. rue St-Denis. 75001 Paris. France

## Description

La présente invention concerne une seringue préremplie à dose unitaire, comportant une carpule ouverte à une de ses extrémités et pourvue d'une zone de section rétrécie voisine de cette extrémité, un organe d'obturation en élastomère engagé dans la carpule, mobile axialement entre une position de stockage et une position d'injection et pourvu d'un circuit intérieur destiné à mettre en communication un embout d'injection avec l'intérieur de la carpule lorsque l'organe d'obturation se trouve dans la position d'injection, ce conduit intérieur comportant un canal axial et un canal radial raccordé au canal axial, cet organe d'obturation étant, au moyen d'un tube central prolongé par un embout de raidissement, solidaire d'une capsule adaptée par-dessus le tronçon d'extrémité ouvert de la carpule et comportant un bouchon d'obturation prolongé par une jupe annulaire souple qui coiffe un embout de raidissement central reliant l'organe d'obturation à la capsule, jupe annulaire à travers laquelle est ménagé le canal radial et qui est équipée d'au moins un bourrelet annulaire périphérique disposé en dessous du canal radial, faisant office de segment de piston pendant l'injection.

L'industrie pharmaceutique, grâce aux bonnes pratiques de fabrication et à des contrôles rigoureux, offre aux malades des médicaments d'une très grande qualité. Cependant, trop souvent encore, cette qualité est hypothéquée par de mauvaises conditions d'administration rencontrées en milieu hospitalier comme en pratique ambulatoire. Pour pallier ces lacunes et faire en sorte que les médicaments soient dispensés, auprès des malades, avec un maximum de sécurité, la dose unitaire a été développée, en particulier pour les formes orales solides et liquides, les formes liquides buvables et les collyres. Pour les médicaments sous forme injectable, les solutions proposées sont encore loin d'être satisfaisantes.

En règle générale, les médicaments injectables sont présentés en ampoules de verre qui offrent des avantages certains pour le fabricant, ne serait-ce que celui d'un coût de production assez faible. Leur utilisation n'est cependant pas dénuée d'inconvénients réels qui tiennent au fait que l'ampoule n'est pas une forme prête à l'emploi. A cet égard, on peut citer les erreurs de volume ou de calcul, la réalisation de mélanges incompatibles dans la même seringue, la contamination particulaire ou microbiologique lors du remplissage de la seringue (souvent réutilisée), la sensibilité du personnel infirmier lors de la préparation de certains principes actifs et l'utilisation de médicaments de manière différée, puisque dans de nombreux services hospitaliers, le remplissage des seringues s'effectue bien avant le moment de l'injection. Enfin, l'identification de la seringue est souvent absente ou incorrecte.

Pour éviter ces difficultés qui peuvent conduire à des accidents graves pour les patients, il existe une seule solution : la généralisation de la dose unitaire qui, seule, permet l'administration directe d'un principe actif sans aucune préparation intermédiaire.

Jusqu'à ce jour, différents types de doses unitaires ont été proposés sous la forme de seringues auto-injectables ou de seringues préremplies. Les difficultés rencontrées lors de leur fabrication ou de leur mise en œuvre n'ont cependant pas permis leur emploi sur une échelle très large.

En outre, le coût total reste un obstacle majeur à leur généralisation. Enfin, certains dispositifs proposés ne donnent pas satisfaction d'une manière complète étant donné qu'ils imposent le percement d'une membrane ou d'un bouchon d'obturation à l'aide d'une double aiguille de fabrication relativement coûteuse et qui risque de surcroît de détacher des particules solides de cette membrane ou de ce bouchon, particules qui peuvent ensuite être drainées par le liquide médicamenteux et injectées avec lui dans le tissu du patient.

Le dispositif qui résout l'ensemble des problèmes énoncés et constitue l'art antérieur le plus proche de la présente invention est décrit dans la demande de brevet européen du même demandeur, publiée sous le N° EP-111 796, qui appartient à l'état de la technique visé au parag. 3 de l'Art. 54 de la CBE.

Dans cette réalisation, la carpule comporte un col de section rétrécie par rapport à son corps et dont la hauteur est relativement importante. L'organe d'obturation est logé dans ce col de section rétrécie pendant toute la période du stockage de la seringue. En raison précisément de ce rétrécissement, l'organe d'obturation en élastomère se trouve fortement comprimé pendant une période de stockage pouvant s'étendre sur plusieurs années et risque de subir une déformation négative, une sorte de rémanence susceptible d'empêcher un retour à l'état initial dans un délai très rapide au moment de l'utilisation de la seringue. Ce phénomène risque de compromettre le fonctionnement normal de l'organe d'obturation et plus particulièrement du bourrelet annulaire périphérique ménagé autour de la jupe annulaire de l'organe d'obturation et disposé en dessous du canal radial. Bien que le remplacement du bourrelet unique par plusieurs lèvres annulaires disposées l'une en dessous de l'autre et un tri préalable des composants permettraient probablement de pallier cet inconvénient, il existe des solutions plus simples qui font l'objet de la présente invention.

En outre, dans ce système de l'art antérieur, la mise en place de l'organe d'obturation engendre une surpression relativement importante à l'intérieur de la carpule. Pour réduire cette surpression qu'il est bénéfique de maintenir à une valeur relativement faible, plusieurs possibilités s'offrent au fabricant : soit prévoir un vide partiel qui est compensé au moment de la mise en place de l'organe d'obturation, soit introduire l'organe d'obturation par pincement pour laisser échapper le gaz, soit prévoir un volume de gaz surmontant le médicament à injecter sensiblement double de celui de l'organe d'obturation. Ces trois solutions sont cependant peu intéressantes, les deux premières en raison de la complexité du coût de l'installation, la troisième en raison de l'augmentation de l'encombrement qu'elle entraîne.

Enfin, un autre problème est celui des tolérances de fabrication de la carpule et de l'organe d'obturation. Etant donné que les tolérances de fabrication de la carpule sont de l'ordre de ± 0.15 mm et celles de l'organe d'obturation de l'ordre de ± 0.10 mm, dans certains cas extrêmes, le serrage au niveau du col est excessif d'où une mise en place difficile de l'organe d'obturation et un frottement important des languettes ou bourrelets annulaires périphériques ménagés en dessous du canal radial, rendant laborieux le déplacement de l'organe d'obturation, agissant comme un piston, à l'intérieur du corps de la carpule. Un tri préalable des carpules et des organes d'obturation en fonction de leur section, leur séparation par lots de mêmes dimensions suivie d'un étiquetage approprié auraient éventuellement pu constituer une solution acceptable à ce problème. Toutefois, une telle solution, bien que réalisable, resterait coûteuse.

La présente invention se propose de pallier tous les inconvénients de l'art antérieur par des moyens constructifs extrêmement simples, qui évitent le recours à des installations complexes et coûteuses.

Dans ce but, la seringue préremplie selon l'invention est caractérisée en ce que le bourrelet annulaire périphérique disposé en dessous du canal radial est positionné en dessous de la zone de section rétrécie de la carpule lorsque l'organe d'obturation se trouve dans ladite position de stockage, de telle manière qu'il ne subisse au plus qu'une faible compression pendant le stockage.

Selon un premier mode de réalisation, la zone de section rétrécie peut être adjacente à l'ouverture de la carpule et le bourrelet annulaire périphérique est dans ce cas localisé à l'extérieur de la carpule lorsque l'organe d'obturation se trouve dans ladite position de stockage.

Selon un second mode de réalisation, la zone de section rétrécie peut être localisée en retrait d'une ceinture de section élargie adjacente à l'extrémité ouverte de la carpule, et le bourrelet annulaire périphérique est dans ce cas disposé dans cette ceinture lorsque l'organe d'obturation se trouve dans ladite position de stockage.

Dans ce dernier cas, le col de la carpule est divisé en deux parties dont l'une, de section réduite, correspond à la zone d'obturation pendant le stockage et dont l'autre, appelée ceinture de section élargie, dont le diamètre sensiblement égal à celui du corps de la carpule permet de localiser le ou les bourrelets annulaires ou les languettes d'étanchéité ménagées en dessous du ou des orifices du canal radial pendant le stockage, en évitant une compression excessive de ce bourrelet et supprimant ainsi le risque d'une déformation permanente. En effet, étant donné que la section de la ceinture élargie est sensiblement égale à celle du corps de la carpule, le bourrelet souple subit pendant le stockage un serrage identique à celui qu'il subit pendant l'injection. Par conséquent, ses propriétés élastiques restent intactes pendant le stockage, ce qui garantit son positionnement quasi instantané après la compression accrue qu'il subit lors de son passage dans la zone de section rétrécie. Le temps de serrage au niveau de cet étranglement étant extrêmement réduit, le bourrelet en élastomère ne peut subir aucune déformation permanente susceptible de mettre en cause le bon fonctionnement du système. Dans la pratique, au moment où intervient ce serrage, l'opérateur dirige l'embout porte-aiguille vers le haut et appuie sur la carpule. Le bourrelet annulaire ménagé en dessous de l'orifice du canal radial dépasse cet étranglement d'un seul coup au cours de la phase de dégazage préliminaire, c'est-à-dire avant que l'aiguille de la seringue ne soit plantée dans le tissu du patient.

Etant donné que la hauteur effective de cette zone d'étranglement est réduite, la diminution du volume de gaz surmontant le médicament liquide à l'intérieur de la carpule ne correspond plus qu'à une fraction du volume de l'organe d'obturation, ce qui résout également le problème de la surpression due à la mise en place dudit organe d'obturation ou celui de l'encombrement de la carpule dont le volume de gaz peut être réduit en conséquence.

Un des avantages, qui découle de l'absence d'une déformation négative due à une certaine perte d'élasticité de la matière élastomère pendant le stockage, est qu'il n'est plus nécessaire de compenser cette déformation en exagérant la dimension du bourrelet ou des lèvres d'étanchéité. Celui-ci peut être fabriqué à sa dimension exacte, le stockage, qu'il dure un mois ou plusieurs années, étant pratiquement sans influence sur la vitesse de réaction de ce bourrelet ou de ces lèvres lorsque l'organe d'obturation est amené en position d'injection. De ce fait, un tri préalable n'est plus nécessaire, ce qui permet une réduction conséquente du coût de la manutention et par suite de la fabrication de ce type de seringue.

Un autre avantage de cette conception résulte de ce que le bourrelet est une protubérance ménagée en dessous de l'orifice du canal radial, se situe pendant le stockage en dessous de l'étranglement et constitue de ce fait un organe de retenue empêchant l'enfoncement accidentel de l'organe d'obturation agissant comme piston à l'intérieur de la carpule. Cette sécurité s'ajoute à la très légère surpression qui règne dans la carpule, due à la mise en place de l'organe d'obturation, et tendant à repousser le piston hors de cette carpule. Pour enfoncer le piston à l'intérieur du corps de la carpule, il faut appuyer volontairement sur son fond, et de préférence imprimer à cette carpule une légère rotation par rapport à la capsule, pour faciliter le passage du bourrelet dans la zone de section rétrécie. Dès que cette zone est dépassée, le serrage du bourrelet à l'intérieur de la carpule est extrêmement faible, ce qui permet de garantir une grande souplesse à l'injection. Ce serrage peut d'ailleurs être calculé au minimum étant donné qu'aucune déformation permanente n'est à craindre pour les raisons expliquées plus en détail ci-dessus.

En outre, du fait que le canal radial est ménagé à l'intérieur d'un matériau élastomère particulièrement mou, ce canal se rétrécit ou s'obture complètement suite à l'écrasement que subit l'organe d'obturation lorsqu'il est soumis à une pression trop importante. Loin de constituer un inconvénient, ce phénomène assure une autorégulation du débit de liquide médicamenteux au moment de l'injection. Par sa conception, ce système impose une vitesse d'injection sensiblement constante et exclut une injection par à-coups ou trop rapide.

La hauteur du bouchon d'obturation de l'organe d'obturation peut être inférieure ou supérieure à la hauteur de la zone de section rétrécie de la carpule. De ce fait, les tolérances de fabrication ne sont plus critiques et l'étanchéité de la seringue pendant le stockage est assurée quel que soit le positionnement dudit bouchon d'obturation par rapport à la surface de ladite zone de section rétrécie, à condition toutefois que l'orifice du canal radial se trouve soit au niveau de ladite zone de section rétrécie, soit en dessous de cette zone.

Selon un mode de réalisation préféré, le diamètre du bouchon d'obturation de l'organe d'obturation est supérieur ou égal au diamètre de la zone de section rétrécie de la carpule et inférieur au diamètre intérieur du corps de la carpule, de manière à assurer l'étanchéité de la carpule lorsque l'organe d'obturation se trouve en position de stockage et à préserver un espace annulaire entre la surface périphérique dudit bouchon d'obturation et la surface intérieure du corps de la carpule lorsque l'organe d'obturation est en position d'injection.

Etant donné que le bouchon d'obturation doit assurer une parfaite étanchéité de la carpule pendant le stockage, il est important que sa paroi périphérique soit fortement en appui contre la surface intérieure de la zone de section rétrécie pendant ce stockage. Pour permettre l'écoulement du médicament pendant l'injection, il est nécessaire que ce bouchon d'obturation ait un diamètre inférieur au diamètre intérieur du corps de la carpule.

Pour faciliter le passage du bourrelet annulaire dans la zone de section rétrécie, la ceinture élargie adjacente à l'extrémité ouverte de la carpule présente avantageusement, au moins sur une partie de sa hauteur, une surface conique dont le diamètre passe progressivement de la valeur du diamètre intérieur maximal de ladite ceinture à celle du diamètre de la zone de section rétrécie.

La surface extérieure de la carpule adjacente à son extrémité ouverte comporte avantageusement un rebord protubérant formant un organe d'accrochage conçu pour coopérer avec un organe d'accrochage complémentaire solidaire de la capsule, et l'organe d'obturation solidaire de la capsule et l'embout de raidissement sont de préférence agencés de telle manière que, lorsque lesdits organes d'accrochage sont engagés l'un dans l'autre, le bouchon d'obturation se trouve en compression et en appui contre la surface de la zone de section rétrécie, et le bourrelet annulaire périphérique se trouve à l'état détendu ou légèrement comprimé dans la ceinture élargie de la carpule. Le positionnement précis de ces organes d'accrochage extérieurs permet d'assurer un positionnement précis des organes intérieurs.

Pour assurer une bonne souplesse à l'organe d'obturation, le bouchon d'obturation et la jupe annulaire ont avantageusement la forme d'un capuchon évidé réalisé en un élastomère mou, dont les parois ont une section telle que la longueur du canal radial soit au plus égale à 5 mm.

L'embout de raidissement, équipé d'un canal axial, comporte avantageusement, à son extrémité supérieure, un évidement annulaire et une entaille sectorielle agencée pour faire communiquer son canal axial avec ledit évidement annulaire. Cette caractéristique constructive autorise une fabrication particulièrement aisée de l'embout de raidissement tout en garantissant une bonne communication entre le canal radial et le canal axial de l'organe d'obturation.

La présente invention, ses principaux avantages et ses caractéristiques essentielles seront mieux compris en référence à la description d'exemples de réalisations et du dessin annexé, dans lequel :

La figure 1 représente une vue en coupe axiale d'une forme de réalisation préférée de la seringue selon l'invention,

La figure 2 représente une vue partielle en coupe de la seringue selon la fig. 1, illustrant en particulier le début de la phase de mise en place de l'organe d'obturation,

La figure 3 représente une vue similaire à celle de la fig. 2, illustrant la position de l'organe d'obturation pendant le stockage de la seringue,

La figure 4 est une vue similaire à celle des fig. 2 et 3, illustrant une position de l'organe d'obturation intermédiaire entre la position de stockage et la position d'injection,

La figure 5 représente une vue similaire à celle des fig. 2 à 4, illustrant l'organe d'obturation en position d'injection,

La figure 6 représente une variante de réalisation de l'organe d'obturation, ce dernier se trouvant en position de stockage, et

La figure 7 représente une autre forme de réalisation de l'organe d'obturation, également illustré en position de stockage.

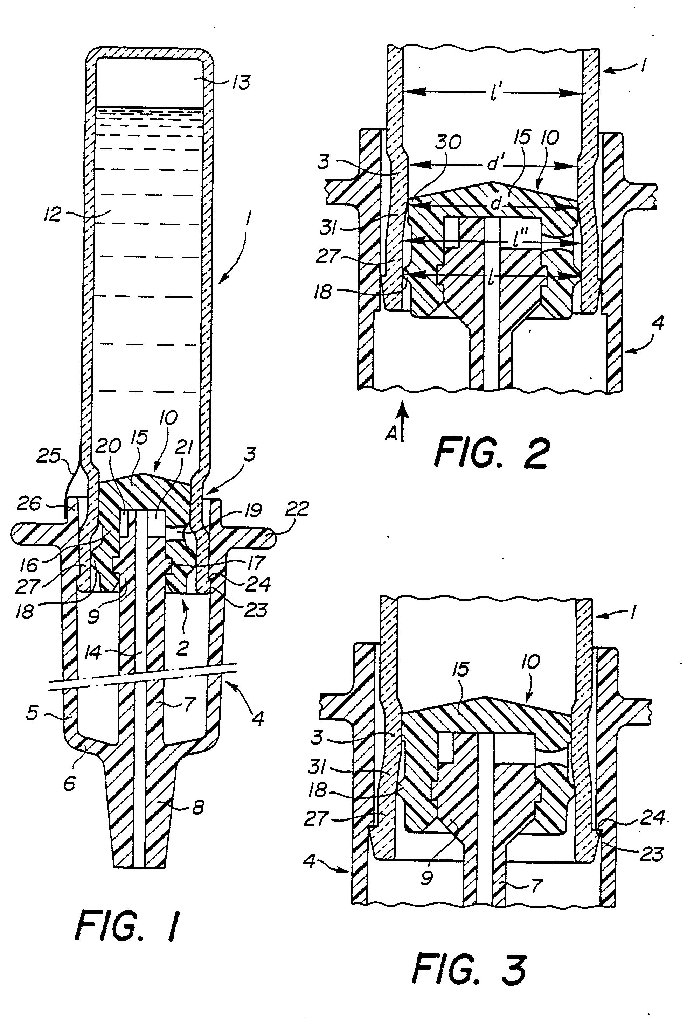

En référence à la fig. 1, la seringue à dose unitaire, représentée dans sa position de stockage, comporte une carpule 1 fermée à une de ses extrémités, munie d'un orifice 2 ménagé à son autre extrémité et pourvu d'une zone 3 de section rétrécie par rapport à son corps disposée au voisinage de cette extrémité ouverte 2. Une capsule creuse 4 est adaptée sur la carpule 1 à son extrémité ouverte 2. Cette capsule se compose d'un capuchon 5 ayant de préférence une paroi latérale cylindrique pleine, mais qui pourrait également être sectorielle, et un fond 6 de préférence plein qui est raccordé à un élément tubulaire central 7. Un embout porte-aiguille 8, également raccordé au fond 6 de la capsule 4, prolonge l'élément tubulaire central 7 dont l'extrémité supérieure forme un embout de raidissement 9 sur lequel est adapté un organe d'obturation souple 10. Cet organe d'obturation 10, comme cela sera décrit plus en détail par la suite, à deux fonctions nettement différentes. La première fonction qu'il exerce pendant le stockage de la seringue consiste à assurer l'étanchéité de la carpule contenant le médicament liquide 12 et une poche de gaz 13 surmontant ce liquide dans la position de la seringue représentée par la figure. La seconde fonction qu'il exerce pendant l'utilisation de la seringue consiste à jouer le rôle d'un piston destiné à refouler le médicament liquide à travers un canal axial 14 ménagé à travers l'élément tubulaire central 7 et l'embout porte-aiguille 8, en direction d'une aiguille (non représentée) pouvant être accouplée par un moyen quelconque à cet embout d'injection.

L'organe d'obturation 10, réalisé en une matière élastomère souple, comporte une partie supérieure en forme de bouchon d'obturation 15 de forme sensiblement cylindrique, prolongée par une jupe annulaire périphérique 16. Cet ensemble est adapté sur l'embout de raidissement 9 auquel la jupe annulaire 16 est liée par des organes de retenue 17. La jupe annulaire 16 est pourvue d'un bourrelet annulaire périphérique 18 ménagé sensiblement à proximité de son extrémité inférieure libre. Un canal radial 19 est ménagé à travers la jupe annulaire 16. Ce canal radial communique avec le canal axial 14 par l'intermédiaire d'un évidement annulaire 20 et d'une entaille sectorielle 21 disposée à l'extrémité supérieure de l'embout de raidissement 9.

Les deux fonctions de l'organe d'obturation 10 sont respectivement remplies par le bouchon d'obturation 15 dont le diamètre est égal ou supérieur au diamètre intérieur de la zone 3 de section rétrécie de la carpule 1 pour assurer une étanchéité parfaite de cette carpule pendant le stockage du médicament liquide 12, et par le bourrelet annulaire périphérique 18 qui fait office de segment de piston pendant la phase d'injection, obligeant ainsi le médicament liquide à pénétrer dans le conduit radial 19 et l'empêchant de s'écouler vers l'orifice de la carpule. Les positions respectives et leur état pour chaque phase d'utilisation de la seringue seront décrits d'une manière plus détaillée en référence aux figures suivantes.

La capsule 4 est avantageusement équipée d'une paire d'ailettes 22 qui facilitent la manipulation de la seringue pendant la phase d'injection.

L'extrémité ouverte 2 de la carpule 1 est de préférence équipée d'un rebord protubérant 23, agencé pour coopérer avec une protubérance 24 solidaire de la surface latérale intérieure du capuchon 5 de la capsule 4. Ces éléments constituent des organes de retenue permettant d'éviter une éjection accidentelle de l'organe d'obturation hors de la carpule en raison de la légère surpression régnant à l'intérieur. Une étiquette, constituée par exemple par une banderole collée à la fois sur la paroi extérieure de la carpule et sur le rebord 26 prolongeant la paroi latérale de la capsule 4 au-delà des ailettes 22, permet de garantir l'inviolabilité de la seringue avant son utilisation finale.

La forme de réalisation illustrée par cette figure montre une carpule 1, dans laquelle la zone 3 de section rétrécie n'est pas directement adjacente à son extrémité ouverte 2, mais est séparée de cette dernière par une ceinture de section élargie 27, dans laquelle est logé le bourrelet annulaire périphérique 18 pendant la phase de stockage illustrée par cette figure.

La fig. 2 illustre le début de l'opération de mise en place de l'organe d'obturation 10 et de la capsule 4 couplée à cet organe d'obturation. Le bouchon d'obturation 15 a un diamètre d égal ou de préférence légèrement supérieur au diamètre d'intérieur de la zone 3 de section rétrécie de la carpule. Dans la version préférée représentée par cette figure, le bord latéral 30 bute contre l'extrémité supérieure de la zone conique 31 qui assure la jonction entre la zone de section rétrécie 3 et la ceinture de section élargie 27 de la carpule 1. A ce stade, le bouchon d'obturation 15 ne subit pratiquement aucune compression si ce n'est une faible contrainte au niveau de son bord latéral 30.

Le diamètre 1 du bourrelet annulaire périphérique 18 a une valeur au moins égale et de préférence légèrement supérieure à la valeur du diamètre 1' de l'intérieur du corps de la carpule 1. En effet, comme mentionné précédemment, ce bourrelet 18 joue le rôle d'un segment de piston lorsque l'organe d'obturation est amené en position d'injection, ce segment de piston devant assurer l'étanchéité avec la paroi de la carpule et empêcher des fuites de médicament liquide vers l'orifice de la carpule. La ceinture de section élargie 27 a de préférence un diamètre 1" égal au diamètre 1' du corps de la carpule. De cette manière, le bourrelet annulaire périphérique 18 se trouve, pendant la phase de stockage, dans un état de faible compression identique à celui ou il se trouvera pendant l'injection, de sorte qu'on évite, comme mentionné précédemment, tous les problèmes de rémanence parasite qui pourraient être provoqués par une compression excessive de ce bourrelet pendant la phase de stockage. Pour supprimer totalement ce risque, il suffirait de prévoir un diamètre 1" de la ceinture 27 supérieur au diamètre 1 du bourrelet 18.

Si l'on continue à enfoncer la capsule et l'organe d'obturation dans le sens de la flèche A, on atteint la position illustrée par la fig. 3. Dans cette position, le bouchon d'obturation 15, dont la hauteur est dans ce cas sensiblement inférieure à la hauteur de la zone 3 de section rétrécie de la carpule 1, ce qui supprime pratiquement tous les risques d'un positionnement erroné dû à un cumul des tolérances de fabrication, est totalement engagé dans cette zone de section rétrécie et se trouve à l'état légèrement, voire fortement comprimé à l'intérieur de cette zone. Une forte compression n'est pas préjudiciable au bon fonctionnement du système puisque la seule fonction de ce bouchon d'étanchéité est d'assurer l'étanchéité de la carpule pendant la phase de stockage. En conséquence, si, par suite d'un stockage particulièrement long, ce bouchon a quelque peu perdu son élasticité et ne retourne pas immédiatement dans son état détendu lorsque l'organe d'obturation pénètre dans le corps de la carpule, ceci ne constitue pas un inconvénient étant donné que le rôle du segment de piston est tenu par le bourrelet annulaire périphérique 18, pour lequel les précautions évitant les phénomènes de rémanence parasitaire ont été prises. La position illustrée par cette figure correspond au stockage de la seringue. Le bourrelet annulaire périphérique est localisé dans la ceinture de section élargie 27 ou éventuellement dans la partie la plus large de la zone conique 31 assurant la jonction entre la zone de section rétrécie 3 et la ceinture de section élargie 27. Le rebord extérieur protubérant 23 de la carpule est en appui contre la protubérance 24 ménagée à l'intérieur de la paroi latérale de la capsule 4. Etant donné que l'organe d'obturation 10 est solidaire de la capsule 4 par l'intermédiaire de l'élément tubulaire central 7 et de l'embout de raidissement 9, le positionnement précis des éléments protubérants 23 et 24 détermine de façon précise la position de l'organe d'obturation 10 à l'intérieur de la carpule pendant la phase de stockage.

En passant de sa position illustrée par la fig. 2 a celle illustrée par la fig. 3, l'organe d'obturation a induit, à l'intérieur de la carpule, une légère surpression due à la diminution du volume à l'intérieur de cette carpule. Cette diminution de volume est égale au produit de la surface de la section de la carpule dans sa zone de section rétrécie par le déplacement subit par l'organe d'obturation entre ces deux positions. Si cette pression induite, qui présente des avantages certains en ce qui concerne une protection contre la contamination accidentelle du médicament contenu dans la carpule, devait être réduite, on pourrait effectuer un remplissage sous vide partiel, de telle manière que l'augmentation de pression soit partiellement absorbée par la dépression due au remplissage sous vide partiel.

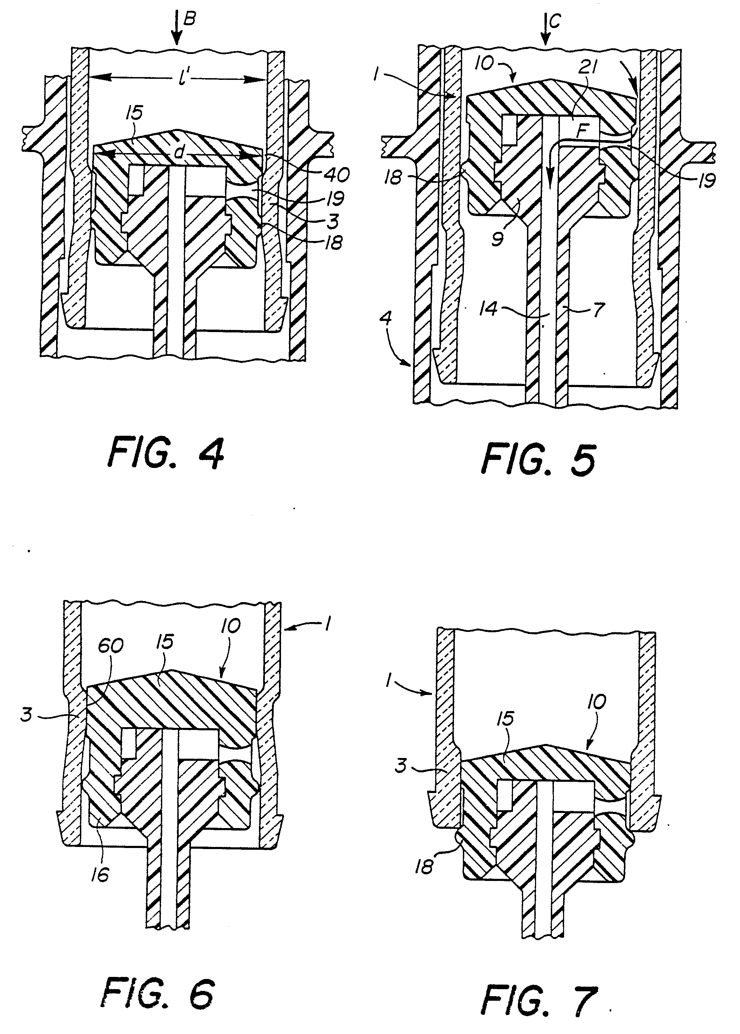

La fig. 4 illustre la phase de préparation de la seringue préliminaire à l'injection. Au cours de cette phase, l'opérateur appuie sur la carpule dans le sens de la flèche B, en ayant bien entendu soin d'orienter cette seringue vers le haut de telle manière que le gaz initialement contenu dans la carpule puisse s'échapper. Cette phase consiste à faire passer le bourrelet annulaire périphérique 18 par-dessus la zone de section rétrécie 3, ce qui le soumet à une compression relativement forte, mais de faible durée. Le bouchon d'obturation 15 a dépassé la zone de section rétrécie 3 et peut se détendre librement. Son diamètre d est inférieur au diamètre 1' du corps de la carpule 1, de telle manière qu'un espace annulaire 40, ménagé entre le flanc latéral de ce bouchon et la paroi intérieure du corps de la carpule, permette l'écoulement du médicament liquide en direction du canal radial 19.

En ce qui concerne le bourrelet annulaire périphérique 18, le passage de la position illustrée par la fig. 3 à celle illustrée par la fig. 4 constitue une étape intermédiaire entre une première position dans laquelle le bourrelet annulaire périphérique 18 joue un rôle essentiellement passif d'organe de retenue empêchant un déplacement accidentel de la carpule dans le sens de la flèche B par rapport à la capsule, et une seconde position dans laquelle il joue un rôle actif de segment de piston qui sera mieux illustré par la fig. 5.

La fig. 5 illustre la seringue dans la position d'injection. Le déplacement de la carpule 1, dans le sens de la flèche C par rapport à la capsule 4 et par rapport à l'organe d'obturation 10 lié à cette capsule, engendre une évacuation du liquide médicamenteux dans le sens de la flèche F à travers le canal radial 19, l'entaille sectorielle 21 pratiquée dans l'organe de raidissement 9 et le canal axial 14 ménagé, à l'intérieur de l'élément tubulaire central 7. Le bourrelet annulaire périphérique 18, qui frotte contre la paroi intérieure du corps de la carpule 1, joue le rôle de segment de piston et force le liquide à suivre le trajet décrit. Du fait que ce bourrelet 18, qui pourrait d'ailleurs être remplacé par une série de lèvres parallèles, soit disposé immédiatement en dessous de l'orifice extérieur du canal radial 19 permet de réduire au minimum le volume mort, c'est-à-dire la quantité de médicament restant dans la seringue après injection.

La fig. 6 illustre une variante dans laquelle la hauteur de la surface cylindrique 60 bordant latéralement à la fois le bouchon d'obturation 15 et la partie supérieure de la jupe annulaire 16 est supérieure à la hauteur de la zone de section rétrécie 3 de la carpule. Cette solution peut présenter de l'intérêt pour éviter que le positionnement de l'organe d'obturation par rapport à la zone de section rétrécie 3 ne soit critique.

L'exemple illustré par la fig. 7 diffère des variantes précédentes en ce que la zone de section rétrécie 3 est adjacente à l'extrémité ouverte de la carpule. Dans ce cas, le bourrelet annulaire périphérique 18 peut être disposé à l'extérieur de la carpule pendant la phase de stockage, de sorte qu'il ne subisse aucune contrainte pendant cette phase tout en remplissant son rôle d'organe de retenue, empêchant une pénétration accidentelle de l'organe d'obturation à l'intérieur de la carpule.

L'exemple de réalisation concrète ci-dessous illustre bien la séparation des fonctions du bouchon d'obturation, d'une part, et du bourrelet annulaire périphérique, d'autre part. Pour une carpule dont le corps a un diamètre intérieur de 10,00 mm, le diamètre intérieur de la zone de section rétrécie est fixé à 9,00 mm, le diamètre du bouchon d'obturation est compris entre 9,20 et 9,80 mm, et le diamètre du bourrelet annulaire d'étanchéité est par exemple égal à 10,25 mm.

Pendant la phase d'injection, le bouchon d'obturation se trouve dans le corps de la carpule. La largeur moyenne, ménagée entre la surface intérieure de la carpule et la surface périphérique du bouchon d'obturation, est comprise entre 0,10 et 0,40 mm. Pour la première valeur, l'écoulement s'effectue normalement et le volume mort, c'est-à-dire la quantité de médicament qui reste dans la carpule en fin d'injection, est très faible. Pour la seconde valeur, l'écoulement s'effectue très bien, mais le volume mort est plus important.

Pendant le stockage, le bouchon d'obturation se trouve dans la zone de section rétrécie. La différence de diamètre entre la zone de section rétrécie et le bouchon d'obturation peut varier entre 0,20 et 0,80 mm. Cette différence doit être absorbée par la compression du bouchon d'obturation. Plus la compression est forte, meilleure est l'étanchéité de la carpule pendant le stockage, mais plus est grande la résistance au décollement au moment de l'utilisation de la seringue.

Compte tenu des dimensions de la zone de section rétrécie et du bourrelet d'étanchéité, la compression de ce dernier, c'est-à-dire sa réduc-·tion de diamètre au moment du passage de l'organe d'obturation de sa position de stockage dans sa position d'injection, est de 1,25 mm. Dans la pratique, on a constaté qu'un serrage de 1,75 mm, dû par exemple à un sous-dimensionnement de la zone de section rétrécie, risque d'engendrer un blocage du système.

Le serrage de 0,25 mm de ce bourrelet, lorsqu'il se trouve dans le corps de la carpule et joue le rôle de segment de piston, est suffisant pour éviter tout écoulement parasite de médicament vers l'ouverture de la carpule.

Dans la pratique, le diamètre du bouchon d'obturation doit être au moins inférieur de 0,10 mm au diamètre intérieur du corps de la carpule et de 0,10 mm supérieur au diamètre de la zone de section rétrécie. Le diamètre du bourrelet annulaire périphérique doit être supérieur d'au moins 0,10 mm au diamètre 30 intérieur du corps de la carpule.

Pour éviter tout problème de mauvais fonctionnement, une solution peut évidemment consister à éliminer les tolérances par un tri approprié des composants. Dans ce cas, on peut fixer les diamètres du corps de la carpule à 10,00 mm, du bouchon d'étanchéité à 9,90 mm, de la zone de section rétrécie de la carpule à 9,80 mm et du bourrelet annulaire à 10,10 mm. La compression au stockage du bouchon d'étanchéité est de 0,10 mm, celle du bourrelet pendant le fonctionnement est de 0,10 mm et la largeur moyenne de l'espace annulaire permettant l'écoulement du liquide est de 0,05 mm.

Si l'on tient compte des tolérances, le diamètre intérieur du corps de la carpule est de 10 ± 0,15 mm, celui du bouchon d'étanchéité est de 9,65 ± 0,10 mm, celui de la zone de section rétrécie de la carpule de 9,35 ± 0,10 mm, et celui du bourrelet annulaire de 10,35 ± 0,10 mm.

Les valeurs de la compression au stockage du bouchon d'étanchéité, de la compression du bourrelet pendant le fonctionnement et de la largeur moyenne de l'espace annulaire varient respectivement entre 0,1 et 0,5 mm ; 0,1 et 0,6 mm ; et 0,05 et 0,30 mm.

Un autre avantage remarquable de ces seringues est la présence d'une légère surpression dans la carpule. Dans la pratique, avec les dimensions fournies ci-dessus, on se propose de maintenir le niveau du liquide à environ 5 mm au-dessous de l'organe d'obturation pendant le stockage. Une distance plus grande supprimerait les avantages de la surpression et augmenterait l'encombrement. La surpression dépend de la réduction de volume induite par la mise en place de l'obturateur. Le déplacement de l'organe d'obturation dépend de la hauteur de la zone de section réduite et de la position initiale occupée par le canal radial. La réduction de volume est proportionnelle à la course de l'organe d'obturation entre sa position de stockage et le début de sa position d'injection. En théorie, on pourrait déterminer la hauteur de gaz, de telle manière qu'en fin de course de l'organe d'obturation, ce gaz ait atteint sa limite d'incompressibilité. On risque cependant le blocage du système par écrasement de l'organe d'obturation.

La hauteur de gaz surmontant le médicament au stockage est déterminée de telle manière que l'opérateur puisse aisément amener l'organe d'obturation dans une position intermédiaire entre la position de stockage et la position d'injection, dans laquelle le canal radial débouche dans le corps de la carpule, dans laquelle le bourrelet annulaire est en place pour assurer sa fonction de segment de piston et dans laquelle le médicament entoure le bouchon d'obturation et l'orifice du canal radial.

Cette position est idéale, car elle permet une mise en place correcte du bourrelet annulaire après son passage dans la zone de section rétrécie.

Si la hauteur de gaz était insuffisante, le liquide risquerait d'atteindre le bourrelet annulaire avant sa mise en place correcte. Pour éviter des risques de fuite, ce bourrelet pourrait être renforcé ou doublé, par exemple sous la forme de deux lèvres annulaires superposées.

Dans la pratique, on prévoit une hauteur de gaz de 8 mm avant la mise en place de l'organe d'obturation. Cette mise en place engendre une réduction de hauteur de 3 mm. Au stockage, la hauteur de gaz est de 5 mm et la pression est de l'ordre de 1,6 bar. Pour amener l'organe d'obturation en position de dégazage, le déplacement requis est de l'ordre de 2 mm, de sorte que la pression du gaz juste avant le dégazage de la seringue atteint 2,6 bars. Un dernier déplacement de 1 mm amène le liquide dans la position idéale mentionnée précédemment.

## Revendications

1. Seringue préremplie à dose unitaire, comportant une carpule, (1) ouverte à une de ses extrémités et pourvue d'une zone de section rétrécie voisine de cette extrémité, un organe d'obturation (10) en élastomère engagé dans la carpule, mobile axialement entre une position de stockage et une position d'injection et pourvu d'un conduit intérieur destiné à mettre en communication un embout d'injection (8) avec l'intérieur de la carpule lorsque l'organe d'obturation se trouve dans la position d'injection, ce conduit intérieur comportant un canal axial et un canal radial raccordé au canal axial, cet organe d'obturation étant, au moyen d'un tube central (7) prolongé par un embout de raidissement (9), solidaire d'une capsule adaptée par-dessus le tronçon d'extrémité ouvert de la carpule et comportant un bouchon d'obturation (15) prolongé par une jupe annulaire souple (16) qui coiffe l'embout de raidissement, jupe annulaire à travers laquelle est ménagé le canal radial et qui est équipée d'au moins un bourrelet annulaire périphérique disposé en dessous du canal radial, caractérisée en ce que le bourrelet annulaire périphérique (18), disposé en dessous du canal radial, faisant office de segment de piston pendant l'injection et positionné en dessous de la zone de section rétrécie (3) de la carpule (1) lorsque l'organe d'obturation (10) se trouve dans ladite position de stockage, de telle manière qu'il ne subisse au plus qu'une faible compression pendant le stockage.

2. Seringue selon la revendication 1, dans laquelle la zone de section rétrécie (3) est adjacente à l'ouverture de la carpule, caractérisée en ce que le bourrelet annulaire périphérique est localisé à l'extérieur de la carpule (1) lorsque l'organe d'obturation se trouve dans ladite position de stockage.

3. Seringue selon la revendication 1, caractérisée en ce que la zone de section rétrécie est localisée en retrait d'une ceinture (27) de section élargie adjacente à l'extrémité ouverte de la carpule, et en ce que le bourrelet annulaire périphérique (18) est disposé dans ladite ceinture lorsque l'organe d'obturation se trouve dans ladite position de stockage.

4. Seringue selon la revendication 1, caractérisée en ce que la hauteur du bouchon d'obturation (15) de l'organe d'obturation (10) est inférieure à la hauteur de la zone de section rétrécie de la carpule.

5. Seringue selon la revendication 1, caractérisée en ce que la hauteur du bouchon d'obturation (15) de l'organe d'obturation (10) est supérieure à la hauteur de la zone de section rétrécie de la carpule.

6. Seringue selon la revendication 1, caractérisée en ce que le diamètre du bouchon d'obturation (15) de l'organe d'obturation (10) est supérieur ou égal au diamètre de la zone de section rétrécie (3) de la carpule et inférieur au diamètre intérieur du corps de la carpule, de manière à assurer l'étanchéité de la carpule lorsque l'organe d'obturation se trouve en position de stockage et à préserver un espace annulaire (40) entre la surface périphérique dudit bouchon d'obturation et la surface intérieure du corps de la capsule lorsque l'organe d'obturation est en position d'injection.

7. Seringue selon la revendication 3, caractérisée en ce que la ceinture élargie (27) adjacente à l'extrémité ouverte de la carpule présente, au moins sur une partie de sa hauteur, une zone conique (31) dont le diamètre passe progressivement de la valeur du diamètre intérieur maximal de ladite ceinture à celle du diamètre de la zone de section rétrécie.

8. Seringue selon la revendication 1, dans laquelle la surface extérieure de la carpule, adjacente à son extrémité ouverte, comporte un rebord protubérant (23) formant un organe d'accrochage conçu pour coopérer avec un organe d'accrochage complémentaire (24) solidaire de la capsule, caractérisée en ce que l'organe d'obturation solidaire de la capsule et l'embout de raidissement (9) sont agencés de telle manière que, lorsque lesdits organes d'accrochage sont engagés l'un dans l'autre, le bouchon d'obturation se trouve au moins partiellement en appui contre la surface intérieure de la zone de section rétrécie et le bourrelet annulaire périphérique se trouve à l'état détendu ou légèrement comprimé.

9. Seringue selon la revendication 1, caractérisée en ce que le canal radial a une longueur au plus égale à 5 mm.

10. Seringue selon la revendication 1, dans laquelle l'embout de raidissement comporte un canal axial (14), caractérisée en ce que l'embout de raidissement est pourvu, à son extrémité supérieure, d'un évidement annulaire (20) et d'au moins une entaille sectorielle (21) agencée pour faire communiquer son canal axial avec ledit évidement annulaire.

## Claims

1. Prefilled single-dose syringe comprising an ampoule (1) open at one of its ends and provided with a narrow-diameter portion neighboring this end, a stoppering device (10) made of an elastomeric material fitted into the ampoule, and being axially movable between a storage position and an injection position, this stoppering device being provided with an interior conduit intented to allow communication between a needle-carrying tip (8) with the interior of the ampoule when the stoppering device is moved into its injection position, this interior conduit comprising an axial channel and a radial channel connected to the axial channel, this stoppering device being integral with a capsule attached to the open ended part of the ampoule by means of a central tube (7) extended with a stiffering tip (9) and comprising a stopper (15) extended with a flexible annular skirt (16) which overlaps the stiffering tip, said skirt being crossed by the radial channel and provided with at least a peripheral annular rim located underneath the radial conduit, acting as a piston segment during the injection phase and located underneath the narrow-diameter portion (3) of the ampoule (1) when the stoppering device (10) is in said storage position, so that it is submitted at most to a low compression during the storage phase.

2. Syringe according to claim 1, in which the narrow-diameter portion (3) is adjacent to the opening of the ampoule, characterized in that the peripheral annular rim is located outside the ampoule (1) when the stoppering device is in said storage position.

3. Syringe according to claim 1, characterized in that the narrow-diameter position is located behind an enlarged-portion ring (27) adjacent to the open end of the ampoule and in that the peripheral annular rim (18) is located within said ring when the stoppering device is in said storage position.

4. Syringe according to claim 1, characterized in that the height of the stopper (15) of the stoppering device (10) is less than the height of the narrow-diameter portion of the ampoule.

5. Syringe according to claim 1, characterized in that the height of the stopper (15) of the stoppering device (10) is greater than the height of the narrow-diameter portion of the ampoule.

6. Syringe according to claim 1, characterized in that the diameter of the stopper (15) of the stoppering device (10) is greater than or equal to the diameter of the narrow-diameter portion (3) of the ampoule and smaller than the inner diameter of the body of the ampoule, so that to assure the tightness of the ampoule when the stoppering device is in storage position and to provide an annular space (40) between the peripheral surface of said stopper and the inner surface of the body of the capsule when the stoppering device is in injection position.

7. Syringe according to claim 3, characterized in that the enlarged ring adjacent to the open end of the ampoule has at least on a part of its height a conical shape (31), the diameter of which varying from the value of the maximum inner diameter of said ring progressively to the value of the diameter of the narrow-diameter portion.

8. Syringe according to claim 1 wherein the outer surface of the ampoule, adjacent to its open end, comprises a protruding rim (23) forming engagement means for cooperating with complementary engagement means (24) integral with the capsule, characterized in that the stoppering device integral with the capsule and the stiffering tip (9) are realized so that the stopper abuts at least partially on the inner surface of the narrow-diameter portion and the peripheral rim is in a slack condition or only under low compression when said engagement means are engaged with each other.

9. Syringe according to claim 1, characterized in that the radial channel has a lenght not greater than 5.00 mm.

10. Syringe according to claim 1, wherein the stiffering tip comprises an axial channel (14), characterized in that the stiffering tip is provided with an annular recess (20) at its upper end and at least one sectorial notch provided to bring into communication its axial channel and said annular recess.


**Patentansprüche**

1. Vorgefüllte Injektionsspritze für einheitliche Dosierung, bestehend aus einer Ampulle (1), die an einem ihrer Enden offen und in der Nähe dieses Endes mit einer Zone von eingeschnürtem Querschnitt versehen ist, weiterhin bestehend aus einem in die Ampulle eingesetzten Verschlußorgan (10) aus Elastomer, welches in axialer Richtung zwischen einer Aufbewahrungsstellung und einer Einspritzstellung axial beweglich und mit einem inneren Verbindungsgang versehen ist, der dazu dient, einen Spritzansatz (8) mit dem Inneren der Ampulle zu verbinden, wenn sich das Verschlußorgan in der Einspritzstellung befindet, wobei diese innere Verbindung einen axialen Kanal und einen an diesen angeschlossenen radialen Kanal aufweist, und dieses Verschlußorgan mit Hilfe eines zentralen durch einen Einspannansatz (9) verlängerten Rohres (7) fest mit einer über das offene Endstück der Ampulle passenden Kapsel verbunden ist, und das einen Verschlußstopfen (15) aufweist, der durch eine ringförmige weiche Schürze (16) verlängert ist und die den Spannansatz überdeckt ; dabei durchdringt der radiale Kanal die Schürze und unter diesem Kanal ist die Schürze mit mindestens einem ringförmigen Wulst auf dem Umfang versehen, welcher während der Injektion als Kolbenring wirkt und der, wenn sich das Verschlußorgan in der Aufbewahrungsstellung befindet, unter der Zone des eingezogenen Querschnitts (3) zu liegen kommt, so daß er während der Aufbewahrung höchstens eine schwache Zusammendrückung erleidet.

2. Spritze nach Anspruch 1, bei welcher die Zone des eingeschnürten Querschnittes (3) an die Öffnung der Ampulle angrenzt, dadurch gekennzeichnet, daß der ringförmige auf dem Umfang verlaufende Wulst außerhalb der Ampulle (1) liegt, wenn sich das Verschlußorgan in der Aufbewahrungsstellung befindet.

3. Spritze nach Anspruch 1, dadurch gekennzeichnet, daß die Zone des eingeschnürten Querschnittes hinter einem Bund (27) mit erweitertem Querschnitt am offenen Ende der Ampulle liegt, und daß der ringförmige auf dem Umfang verlaufende Wulst (18) im Bereich dieses Bundes liegt, wenn sich das Verschlußorgan in der Aufbewahrungsstellung befindet.

4. Spritze nach Anspruch 1, dadurch gekennzeichnet, daß die Höhe des Verschlußstopfens (15) des Verschlußorgans (10) kleiner ist als die Höhe der Zone an der Ampulle mit eingeschnürtem Querschnitt.

5. Spritze nach Anspruch 1, dadurch gekennzeichnet, daß die Höhe des Verschlußstopfens (15) des Verschlußorgans (10) größer ist als die Zone an der Ampulle mit eingeschnürtem Querschnitt.

6. Spritze nach Anspruch 1, dadurch gekennzeichnet, daß der Durchmesser des Verschlußstopfens (15) des Verschlußorgans (10) größer oder gleich dem Durchmesser der Zone der Ampulle mit eingeschnürtem Querschnitt (3) ist und kleiner als der Durchmesser des Ampullenschaftes, um die Dichtheit der Ampulle zu gewährleisten, wenn sich das Verschlußorgan in Aufbewahrungsstellung befindet und um einen Ringraum (40) zwischen der Umfangsfläche des Verschlußstopfens und der Innenfläche der Kapsel (Ampulle) zu belassen, wenn sich das Verschlußorgan in Einspritzstellung befindet.

7. Spritze nach Anspruch 3, dadurch gekennzeichnet, daß der erweiterte Bund (27) am offenen Ende der Ampulle mindestens auf einem Teil seiner Höhe eine konische Zone (31) aufweist, deren Durchmesser allmählich von dem Wert des maximalen Innendurchmessers des Bundes auf den des Durchmessers der Zone mit eingeschnürtem Querschnitt übergeht.

8. Spritze nach Anspruch 1, bei welcher die Außenfläche der Ampulle, an ihrem offenen Ende, einen vorspringenden Rand (23) aufweist, der ein Festhalteorgan bildet und vorgesehen ist, um mit einem entsprechenden Festhalteorgan (24) an der Kapsel zusammenzuwirken, dadurch gekennzeichnet, daß das mit der Kapsel verbundene Verschlußorgan und der Spannansatz (9) so ausgeführt sind, daß sich der Verschlußstopfen mindestens teilweise gegen die Innenfläche der Zone mit eingeschnürtem Querschnitt legt und der ringförmige auf dem Umfang verlaufende Wulst sich in entspanntem oder leicht zusammengedrücktem Zustand befindet, wenn die Festhalteorgane ineinandergreifen.

9. Spritze nach Anspruch 1, dadurch gekennzeichnet, daß der radiale Kanal eine Länge von höchstens 5 mm hat.

10. Spritze nach Anspruch 1, bei welcher der Spannansatz einen axialen Kanal (14) aufweist, dadurch gekennzeichnet, daß der Spannansatz an seinem oberen Ende mit einer ringförmigen Aussparung (20) oder zumindest mit einer sektorförmigen Ausklinkung (21) versehen ist, die dazu dient, seinen axialen Kanal mit der ringförmigen Aussparung in Verbindung zu bringen.

**FIG. 1**

**FIG. 2**

**FIG. 3**

1

FIG. 4

FIG. 5

FIG. 6

FIG. 7